# EUROPÄISCHE PATENTSCHRIFT

(11) **EP 1 397 662 B1**
(45) Veröffentlichungstag und Bekanntmachung des Hinweises auf die Patenterteilung: **24.01.2007**
(21) Anmeldenummer: 02747400.6
(22) Anmeldetag: 17.06.2002
(51) Int. Cl.: G01N 1/20, G01N 33/04, A01J 5/04, G01F 1/00

(54) **VERFAHREN UND VORRICHTUNG ZUR ENTNAHME EINER PROBE AUS EINER FLUIDCHARGE**
METHOD AND DEVICE FOR WITHDRAWING A SAMPLE FROM A FLUID BATCH
PROCEDE ET DISPOSITIF DE PRELEVEMENT D'UN ECHANTILLON DANS UNE CHARGE FLUIDIQUE

(30) Priorität: 18.06.2001 DE 10129246
(43) Veröffentlichungstag der Anmeldung: 17.03.2004
(73) Patentinhaber: Schwarte Logistic GmbH, 59227 Ahlen (DE)
(72) Erfinder: BÖHM, Alfred, 94234 Viechtach (DE); BARLIAN, Reinhold, A., 97980 Bad Mergentheim (DE)
(74) Vertreter: Heim, Hans-Karl
(86) Internationale Anmeldenummer: PCT/EP2002/006656
(87) Internationale Veröffentlichungsnummer: WO 2002/103324

(56) Entgegenhaltungen:
- EP-A- 0 217 155
- EP-A- 0 223 190
- EP-A- 0 872 175
- DE-A- 4 343 717
- GB-A- 1 490 082
- US-A- 3 318 155
- US-A- 5 121 773
- US-A- 5 645 012

## Beschreibung

Die Erfindung betrifft ein Verfahren zur Entnahme einer Probe aus einer Fluidcharge, insbesondere zur Entnahme einer repräsentativen Probe aus einer Milchcharge. Ferner betrifft die Erfindung eine Vorrichtung zur Entnahme einer Probe aus einer Fluidcharge.

Aus der EP O 709 666 B1 ist ein Verfahren und eine Vorrichtung zur Entnahme einer Probe aus einer Fluidcharge bekannt, bei dem bzw. bei der eine Teilmenge einer durch eine Leitung in einen Tank einströmenden Fluidcharge mit Hilfe einer Pumpe in einen Zwischenbehälter gefördert wird. Aus dem Zwischenbehälter wird nach dem Befüllen mit der Teilmenge eine vorgegebene Probenmenge entnommen und der Zwischenbehälter anschließend entleert.

Die EP 0 617 789 B1 beschreibt ein Verfahren und eine Vorrichtung zur Entnahme einer Probe aus einer Milchcharge, bei dem mit Hilfe einer Pumpe eine vorgegebene Probenmenge unmittelbar aus der an der Entnahmestelle vorbeiströmenden Fluidcharge entnommen wird. Die Förderleistung der Pumpe wird dabei in Abhängigkeit von der Strömungsgeschwindigkeit und dem Füllgrad der Leitung geregelt.

Die DE 100 10 187 A1 offenbart eine Probeentnahmevorrichtung in einer Milchsammelanlage, bei der zwei Entnahmestellen mit unterschiedlichen Strömungsquerschnitten vorgesehen sind, die jeweils mit einem separaten Zwischenbehälter verbunden sind. In Abhängigkeit von der Gesamtmenge der Fluidcharge strömen unterschiedliche Mengen durch die beiden Entnahmestellen in die Zwischenbehälter, aus denen anschließend vorgegebene Probenmengen der Fluidcharge entnommen werden können.

Die DE 38 23 791 A1 offenbart ein Verfahren und eine Vorrichtung zur Gewinnung von Flüssigkeitsproben, insbesondere von Milchproben, bei dem bzw. bei der aus einer zwischen einem Anlieferungsbehälter und einem Sammelbehälter vorgesehenen Förderleitung entsprechend einem vorgegebenen Proportionalitätsfaktor ein konstanter Teilstrom der durch die Förderleitung strömenden Fluidcharge in einen Zwischenbehälter gefördert wird. Der Proportionalitätsfaktor wird vor dem Befüllen in Abhängigkeit von einer angenommenen Gesamtmenge der zu sammelnden Fluidcharge eingestellt. Nach dem Ende des Befüllens des Sammelbehälters wird die Probe aus dem Zwischenbehälter entnommen.

Aus der DE 85 28 090 Ul ist eine Vorrichtung zur Probenentnahme bekannt, bei der an der Förderleitung für die Fluidcharge zwei Abzweigleitungen vorgesehen sind, wobei jede Abzweigleitung mit einem Probeentnahmebehälter verbunden ist. Durch die Abzweigleitungen strömen Teilmengen entsprechend unterschiedlicher, durch die Strömungsquerschnitte der Abzweigleitungen vorgegebener Proportionalitätsfaktoren in die beiden Probeentnahmebehälter. In Abhängigkeit von der erwarteten Gesamtmenge der Fluidcharge werden entweder nur eine der beiden Abzweigleitungen oder beide Abzweigleitungen geöffnet und die Probebehälter befüllt, aus denen anschließend die Probe entnommen wird.

In der DE 35 28 827 A1 ist eine Probeentnahmevorrichtung beschrieben, bei der entsprechend einer Vortagesliefermenge aus der aktuellen Liefermenge eine vorgegebene Probenmenge entnommen wird. Dabei werden die Förderimpulse entsprechend der Vortagesliefermenge bestimmt und auf die aktuelle Liefermenge gleichmäßig verteilt.

Aus der DE 35 02 858 A1 sowie aus der EP 0 078 828 B1 ist jeweils eine Vorrichtung zur Entnahme von Milchproben bekannt, bei der eine Probenmenge der Fluidcharge unmittelbar aus der Fluidcharge in einen Probenbehälter gefüllt wird. Die Probenmenge wird hierzu in Abhängigkeit von einer geschätzten Gesamtmenge der Fluidcharge in eine vorgegebene Anzahl Teilmengen unterteilt, die aus der Fluidcharge entnommen werden. In Abhängigkeit von dem tatsächlichen Förderstrom der Fluidcharge werden dann die Pausenzeiten zwischen den zu entnehmenden Teilmengen berechnet.

Aus der US 5,645,012 A ist ein Verfahren und eine Vorrichtung zur Milchabnahme und dem Ziehen einer Probe bekannt. Die Probennahme wird auch über einen vorgeschalteten Volumenstromsensor gesteuert. Auf größere Schwankungen von zu übernehmender Milch ist man jedoch nicht eingestellt, so dass die gezogene Probe in vielen Fällen als nicht repräsentativ angesehen werden muss.

Die in der EP 0 217 155 A1 beschriebene Vorrichtung zur Milchübernahme zeigt zwar einen Zwischenbehälter mit Rührwerk, wobei die Probe aus dem Zwischenbehälter gezogen wird. Die Probleme der Restmilch und der Verschleppung von Probenmilch in eine nächste Probe sind jedoch darin nicht berücksichtigt worden.

Aus der EP 0 223 190 A2 ist ein Verfahren zur Gewinnung von Flüssigkeitsproben, insbesondere Milchproben, bekannt. Hierbei wird aus der Förderleitung in einen Sammelbehälter, beispielsweise Milch, abgezweigt. Ist der Sammelbehälter voll, so wird bei gleichzeitigem Abführen von Milch aus dem Sammelbehälter weiter Milch in den Sammelbehälter geleitet. Diese weitere Einleitung findet diskontinuierlich durch ein Takten eines entsprechenden Ventiles statt, wobei sich der Proportionalitätsfaktor ständig ändert.

Diesen bekannten Verfahren bzw. Vorrichtungen ist gemeinsam, dass zwar eine definierte Probenmenge aus der Fluidcharge entnehmbar ist, eventuelle Schwankungen in der Zusammensetzung der Fluidcharge bei der Entnahme der Probe jedoch allenfalls unzureichend miterfaßt werden, so dass die Zusammensetzung der Probe kein tatsächliches prozentuales Abbild der Inhaltsstoffe der zu prüfenden Fluidcharge darstellt.

Der Erfindung liegt nun die **Aufgabe** zugrunde, ein Verfahren bzw. eine Vorrichtung zur Entnahme einer Probe aus einer Fluidcharge anzugeben, bei dem bzw. mit der eine vorgegebene Probenmenge entnehmbar ist, die in ihrer Zusammensetzung zumindest annähernd der tatsächlichen Zusammensetzung der zu prüfenden Fluidcharge entspricht und für diese repräsentativ ist.

Die Aufgabe wird durch ein Verfahren mit den Merkmalen nach Anspruch 1 gelöst. Ferner wird die Aufgabe durch eine Vorrichtung mit den Merkmalen nach Anspruch 12 gelöst. Weitere vorteilhafte Weiterbildungen der Erfindung ergeben sich aus der nachfolgenden Beschreibung, der Zeichnung sowie den jeweils abhängigen Ansprüchen.

Bei der Erfindung wird kontinuierlich eine Teilmenge des Volumenstroms der Fluidcharge in den Zwischenbehälter gefördert. Da der Volumenstrom der Fluidcharge betrachtet über die Zeit Schwankungen unterliegt, die beispielsweise durch Schwankungen in der Förderleistung der Pumpe, durch Ansaugen von Luft in den Volumenstrom oder Ähnliches verursacht sind, schlägt die Erfindung vor, die Teilmenge in Abhängigkeit von dem aktuellen Volumenstrom entsprechend einem vorgegebenen Proportionalitätsfaktor während der Entnahme zu verändern.

Auf diese Weise wird erreicht, dass einerseits die auftretenden Schwankungen im Volumenstrom der Fluidcharge und andererseits die im Volumenstrom variierende Zusammensetzung der Fluidcharge, die insbesondere bei Emulsionen wie Milch auftritt, bei der Teilmenge anteilsmäßig mitberücksichtigt sind. Durch die gezielte volumenstromproportionale Förderung der Teilmenge in den Zwischenbehälter, die in Abhängigkeit von dem aktuell erfassten Volumenstrom eingestellt wird, zeigt die Teilmenge und damit insbesondere auch die aus der Teilmenge zu entnehmende Probenmenge, die mit vorgegebenen Volumen oder Gewicht aus der Teilmenge entnommen wird, eine Zusammensetzung, die repräsentativ für die Zusammensetzung der Fluidcharge ist, also ein prozentuales Abbild der Inhaltsstoffe der Fluidcharge enthält.

Als Proportionalitätsfaktor, mit dem das Verhältis zwischen dem aktuellen Volumenstrom der Fluidcharge und der aus der Fluidcharge entnommenen Teilmenge definiert ist, wird vorzugsweise ein Verhältnis vorgegeben, bei dem sichergestellt ist, dass die aus der Fluidcharge entnommene Teilmenge zumindest gleich, vorzugsweise aber größer ist als die vorgegebene Probenmenge. Auf diese Weise wird gewährleistet, dass auch bei großen mengenmäßigen Unterschieden zwischen verschiedenen Fluidchargen die in den Zwischenbehälter geförderte Teilmenge so groß ist, dass jederzeit die vorgegebene Probenmenge entnehmbar ist.

Da es bei den Gesamtmengen zwischen den einzelnen Fluidchargen häufig zu großen Schwankungen kommt, bei der Annahme von Milch können die abzunehmenden Milchchargen beispielsweise in einem Bereich von 10 Litern bis zu 10.000 Litern liegen, muß sichergestellt sein, dass jederzeit eine repräsentative Probenmenge abgegeben werden kann.

Zum Bestimmen des Proportionalitätsfaktors wird bei einer bevorzugten Variante des Verfahrens deshalb vorgeschlagen, die Gesamtmenge der Fluidcharge, aus der die Probe entnommen werden soll, zunächst zu schätzen. Diese Schätzwerte werden üblicherweise für nachfolgende Probenentnahmen individualisiert für die jeweiligen Annahmestelle abgespeichert. Anschließend wird aus der geschätzten Gesamtmenge der Fluidcharge und der vorgegebenen Probenmenge der vorzugebende Proportionalitätsfaktor bestimmt. Durch diese Methode zum Bestimmen des Proportionalitätsfaktors wird sichergestellt, dass auch bei großen Differenzen zwischen den zu erwartenden Gesamtmengen der verschiedenen Fluidchargen jederzeit eine ausreichend große Teilmenge in den Zwischenbehälter gefördert werden kann, um aus dieser Teilmenge die vorgegebene Probenmenge entnehmen zu können.

Um sicher zu gehen, dass auch bei einer ungenauen Schätzung der Gesamtmenge der jeweiligen Fluidcharge einerseits eine ausreichend große Teilmenge im Zwischenbehälter enthalten ist, der Zwischenbehälter andererseits jedoch nicht überfüllt wird, ist es ferner von Vorteil, den vorgebbaren Proportionalitätsfaktor so einzustellen, dass die sich aus der geschätzten Gesamtmenge und dem Proportionalitätsfaktor rechnerisch ergebende Teilmenge einem Vielfachen der vorgegebenen Probenmenge entspricht, jedoch um ein Mehrfaches unter der in den Zwischenbehälter füllbaren Maximalmenge liegt.

Das erfindungsgemäße Verfahren kann insbesondere für phasenbildende oder inhomogene Fluide, beispielsweise in der Lebensmittelindustrie, eingesetzt werden. Besonders bevorzugt wird das erfindungsgemäße Verfahren für Flüssigkeiten und/oder Emulsionen verwendet, die während des Förderns zu einem Aufschäumen neigen, wodurch der Volumenstrom der Fluidcharge großen Schwankungen unterliegt. Insbesondere wird das erfindungsgemäße Verfahren zur repräsentativen Probeentnahme aus Milchchargen eingesetzt.

Handelt es sich bei dem zu überprüfenden Fluid um eine schäumende Flüssigkeit oder eine Emulsion, wird bei einer besonders bevorzugten Verfahrensvariante vorgeschlagen, die im Zwischenbehälter enthaltene Teilmenge der Fluidcharge vor der Probenentnahme homogen zu durchmischen. Durch das homogene Durchmischen wird erreicht, dass die Teilmenge im Zwischenbehälter eine besonders gleichmäßige Verteilung der Inhaltsstoffe aufweist und gleichzeitig auch in ihrer Konsistenz der Fluidcharge entspricht, so dass insbesondere auch die aus der Teilmenge zu entnehmende Probenmenge sowohl in ihrer Zusammensetzung als auch in ihrer Konsistenz für die zu prüfende Fluidcharge repräsentativ ist. Dies ist insbesondere bei der Prüfung und Qualitätsbeurteilung von Emulsionen, wie Milch oder Sahne, von besonderem Vorteil.

Zum Durchmischen der Teilmenge wird insbesondere vorgeschlagen, im Zwischenbehälter einen Unterdruck zu erzeugen, der anschließend ausgeglichen wird. Dabei kann insbesondere beim Ausgleichen des Unterdrucks die im Zwischenbehälter enthaltene Teilmenge belüftet werden, damit die Teilmenge durch die einströmende Luft homogen durchmischt wird und die Inhaltsstoffe in der Teilmenge gleichmäßig verteilt sind. Des Weiteren wird bei dieser Verfahrensvariante insbesondere vorgeschlagen, die Probe unmittelbar nach dem homogenen Durchmischen zu entnehmen, indem die Probenmenge aus dem Zwischenbehälter direkt in einen Probenbehälter abgegeben wird. Auf diese Weise wird erreicht, dass die in den Probenbehälter gefüllte Probenmenge repräsentativ für die Fluidcharge ist, aus der die Teilmenge zuvor entnommen worden war.

Um ein besonders genaues Befüllen des Zwischenbehälters mit der Teilmenge zu erreichen, wird ferner vorgeschlagen, den Innendruck im Zwischenbehälter zumindest während des Befüllens mit der Teilmenge auszugleichen, damit eventuelle Druckschwankungen im Zwischenbehälter, die während des Befüllens entstehen, ausgeglichen werden, wodurch eine sehr exakte volumenstromproportionale Entnahme der Teilmenge aus der Fluidcharge möglich wird.

Des Weiteren wird bei einer besonders bevorzugten Verfahrensvariante der Zwischenbehälter vor dem Befüllen mit der Teilmenge zunächst durch Fluid aus der Fluidcharge, aus der die Probe nachfolgend entnommen werden soll, vorgespült. Auf diese Weise wird erreicht, dass noch eventuell im Zwischenbehälter anhaftende Reste aus einer Fluidcharge, aus der zuvor eine Probe entnommen worden war, aus dem Zwischenbehälter gespült werden und eine Verfälschung der Probe durch eventuell verschleppte Reste aus der vorherigen Fluidcharge wirksam verhindert ist. Bei dieser Verfahrensvariante wird ferner vorgeschlagen, die Förderleistung, mit der die Teilmenge nach dem Vorspülen in den Zwischenbehälter gefördert wird, gegenüber der zum aktuellen Volumenstrom proportialen Förderleistung kurzzeitig überproportional zu erhöhen. Das an der Entnahmestelle aus der vorbeiströmenden Fluidcharge abgeführte Fluid wird dabei so lange überproportional in den Zwischenbehälter gefördert, bis die geförderte Menge zumindest annähernd zu dem Volumenstrom proportional ist, der die Entnahmestelle bei Erreichen der Proportionalität bereits passiert hat. Durch die kurzfristige überproportionale Förderleistung wird erreicht, dass die insgesamt am Ende des Entnahmevorgangs aus der Fluidcharge im Zwischenbehälter enthaltene Teilmenge zumindest annähernd proportional zur Gesamtmenge der Fluidcharge ist, obwohl vor der eigentlichen Entnahme ein Teil der Fluidcharge zum Vorspülen des Zwischenbehälters verwendet wurde und demzufolge bei der proportionalen Förderung der Teilmenge in den Zwischenbehälter nicht miterfasst werden konnte.

Zum Entleeren des Zwischenbehälters nach der Probenentnahme ist es ferner von Vorteil, die im Zwischenbehälter enthaltene Restmenge durch Erzeugen eines Überdrucks im Zwischenbehälter, beispielsweise mit Hilfe eines im Zwischenbehälter beweglichen Kolbens, aus dem Zwischenbehälter zu entfernen.

Des Weiteren ist es vorteilhaft, wenn nach der Probenentnahme und/oder nach dem Entleeren des Zwischenbehälters in diesem ein Unterdruck erzeugt wird, durch den ein Reinigungsgas, insbesondere Luft, durch mit dem Zwischenbehälter in Verbindung stehende Leitungen in den Zwischenbehälter gesaugt wird. Auf diese Weise wird erreicht, dass die Leitungen, durch die zuvor Fluid in den Zwischenbehälter eingeströmt bzw. Fluid aus dem Zwischenbehälter in den Probenbehälter abgefüllt wurde, von noch eventuell in den Leitungen anhaftenden Resten des Fluides gereinigt werden.

Des Weiteren wird vorgeschlagen, die Rückführleitung, durch die der Zwischenbehälter entleert wird, von Fluidresten zu reinigen, indem im Zwischenbehälter nach dem Ansaugen des Reinigungsgases ein Überdruck erzeugt und das Reinigungsgas durch die Rückführleitung aus dem Zwischenbehälter abgeleitet wird.

Handelt es sich bei dem Fluid, aus dem eine Probe entnommen werden soll, um eine schäumende Flüssigkeit, wie Milch, die für nachfolgende Verfahrensschritte entgast werden muss, wird ferner vorgeschlagen, die Teilmenge vor dem Entgasen der Fluidcharge aus dieser zu entnehmen.

Gemäß einem weiteren Aspekt betrifft die Erfindung eine Vorrichtung zur Entnahme einer Probe aus einer Fluidcharge. Bei der erfindungsgemäßen Vorrichtung ist in der Leitung, durch die die Fluidcharge gefördert wird, mindestens ein Volumenstromsensor vorgesehen, mit dem der Volumenstrom der Fluidcharge erfaßbar ist. Der Volumenstromsensor ist mit einer Steuereinrichtung verbunden, die ihrerseits mit einer Fördereinrichtung in Verbindung steht, mit der eine Teilmenge aus der durch die Leitung strömenden Fluidcharge in einen Zwischenbehälter gefördert wird. Die Steuereinrichtung stellt in Abhängigkeit von dem durch den Volumenstromsensor erfassten Volumenstrom die Förderleistung der Fördereinrichtung so ein, dass die in den Zwischenbehälter zu fördernde Teilmenge zumindest annähernd proportional zu dem von dem Volumenstromsensor aktuell erfassten Volumenstrom ist.

Um eine möglichst kompakte Anordnung der Vorrichtung zu erreichen, die sich durch ein vergleichsweise geringes Bauvolumen auszeichnet, wird ferner vorgeschlagen, die Fördereinrichtung zumindest teilweise in den Zwischenbehälter zu integrieren, wobei die Fördereinrichtung zur Erzeugung eines Unterdrucks und eines Überdrucks im Zwischenbehälter ausgelegt ist. Auf diese Weise kann mit Hilfe der Fördereinrichtung der Zwischenbehälter durch den erzeugten Unterdruck gefüllt, bzw. durch den erzeugten Überdruck entleert werden. Durch ein wechselweises Erzeugen von Unterdruck und sich daran anschließendes Ansaugen von Luft in den Zwischenbehälter kann das im Zwischenbehälter enthaltene Fluid gezielt durchgemischt werden.

Bei einer besonders bevorzugten Ausführungsform der erfindungsgemäßen Vorrichtung weist die Fördereinrichtung einen im Zwischenbehälter beweglichen Kolben auf, durch den der Zwischenbehälter auf elegante Weise definiert gefüllt und entleert werden kann, wobei durch eine entsprechende Ansteuerung des Kolbens ein sehr exaktes, volumenstromproportionales Befüllen des Zwischenbehälters möglich ist.

Des Weiteren ist es von Vorteil, wenn die aus dem Zwischenbehälter abgebbare Probenmenge proportional zum Hub des Kolbens ist, so dass unabhängig vom Füllstand des Zwischenbehälters jederzeit mengenmäßig zumindest annähernd identische Probenmengen unterschiedlicher Fluidchargen definiert abgegeben werden können. Hierbei ist es besonders vorteilhaft, wenn die abgebbare Probenmenge direkt proportional zum Hub des Kolbens ist.

Um das Anhaften von Restfluid im Zwischenbehälter zumindest weitestgehend zu vermeiden, wird bei einer bevorzugten Weiterbildung der Fördereinrichtung mit Kolben vorgeschlagen, zumindest an den Abschnitten des Zwischenbehälters und/oder des Kolbens, die mit dem Fluid in unmittelbaren Kontakt kommen können, hydrophobe und/oder polierte Oberflächen auszubilden, so dass sich eventuell an den Oberflächen anhaftendes Fluid sammelt und aus dem Zwischenbehälter abfließt.

Des Weiteren wird bei einer bevorzugten Ausführungsform der erfindungsgemäßen Vorrichtung vorgeschlagen, zum Druckausgleich des Zwischenbehälters eine Druckausgleichsleitung vorzusehen, um den im Zwischenbehälter wirkenden Druck gegenüber dem Umgebungsdruck auf einfache Weise schnell ausgleichen zu können. Bei Verwendung einer derartigen Druckausgleichsleitung ist es ferner von Vorteil, wenn der Druckausgleich durch ein vorzugsweise von der Steuereinrichtung ansteuerbares Stellventil in der Druckausgleichsleitung regelbar ist, so dass, sofern dies gewünscht ist, der Druckausgleich im Zwischenbehälter geregelt vorgenommen oder gegebenenfalls verhindert werden kann.

Ferner wird für eine besonders kompakte Bauweise der erfindungsgemäßen Vorrichtung vorgeschlagen, den Zwischenbehälter und die Probeentnahmeeinrichtung durch eine gemeinsame, absperrbare Rohrverzweigung miteinander zu verbinden. Zum Befüllen und Entleeren des Zwischenbehälters weist die Rohrverzweigung bei dieser Ausführungsform ferner eine Einströmleitung und eine Rückführleitung auf, durch die die Rohrverzweigung mit der Leitung für die Fluidcharge verbindbar ist.

Bei einer besonders bevorzugten Weiterbildung dieser Ausführungsform der erfindungsgemäßen Vorrichtung wird ferner vorgeschlagen, in der Rohrverzweigung ein insbesondere von der Steuereinrichtung betätigbares Mehrwegventil vorzusehen, mit dem durch unterschiedliche Schaltzustände der Zwischenbehälter befüllbar und entleerbar sowie mit der Probeentnahmeeinrichtung verbindbar ist.

Nachfolgend wird die Erfindung anhand eines Ausführungsbeispiels unter Bezugnahme auf die Zeichnung näher erläutert. Darin zeigen:
- Fig. 1: eine schematische Seitenansicht der erfindungsgemäßen Vorrichtung zur Entnahme einer Probe aus einer Milchcharge in teilweise geschnittener Ansicht;
- Fig. 2: eine schematische Schnittansicht eines mit der Vorrichtung nach Fig. 1 verwendeten Zwischenbehälters mit integrierter Fördereinrichtung; und
- Fig. 3a bis 3d: verkleinerte schematische Schnittansichten des Zwischenbehälters nach Fig. 2 in unterschiedlichen Betriebsstellungen.

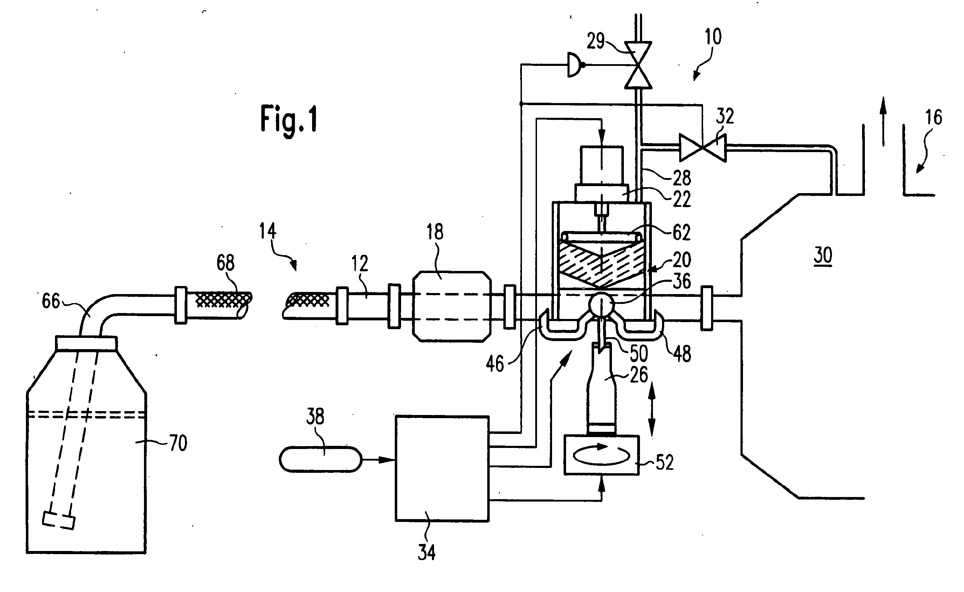

In Fig. 1 ist in schematischer Seitenansicht eine Vorrichtung 10 zur Entnahme einer Probe aus einer Milchcharge gezeigt. Die Vorrichtung 10 ist in eine Leitung 12 einer Milchförderanordnung 14 einer stationären oder verfahrbaren Tankanlage 16 integriert und in Milchförderrichtung in die Tankanlage 16 gesehen stromaufwärts vor einem gegebenenfalls in der Milchförderanordnung 14 vorgesehenen Gasmeßverhüter oder Luftabscheider positioniert.

Die Vorrichtung 10 weist einen Volumenstromsensor 18 auf, der in Milchförderrichtung längs der Leitung 12 gesehen stromaufwärts vor und unmittelbar benachbart zu einem Zwischenbehälter 20 angeordnet ist, dessen Aufbau später noch erläutert wird. An dem Zwischenbehälter 20 ist eine Fördereinrichtung 22 vorgesehen, mit der der Zwischenbehälter 20 befüllt und entleert werden kann. Unter dem Zwischenbehälter 20 ist eine Probeentnahmeeinrichtung 24 vorgesehen, in der ein Probenbehälter 26 mit einer vorgegebenen Probenmenge aus dem Zwischenbehälter 20 befüllbar ist.

Der Innenraum des Zwischenbehälters 20 ist mit einer nahe der Fördereinrichtung 22 an der Oberseite des Zwischenbehälters 20 abzweigenden Druckausgleichsleitung 28 verbunden, die durch ein erstes Stellventil 29 mit der Umgebung und durch ein zweites Stellventil 32 mit einem Luftabscheider 30 der Tankanlage 16 verbunden werden kann. Die beiden Stellventile 29 und 32 dienen zum Ausgleich des im Zwischenbehälter 20 wirkenden Drucks, wie später noch erläutert wird.

Die Vorrichtung 10 weist ferner eine Steuereinrichtung 34 auf, die durch elektrische Leitungen mit dem Volumenstromsensor 18, mit der Fördereinrichtung 22, mit der Probeentnahmeeinrichtung 24, mit den Stellventilen 29 und 32 sowie mit einem am Zwischenbehälter 20 vorgesehenen Mehrwegventil 36, dessen Aufbau später noch erläutert wird, zur Signalübertragung verbunden ist. Die Steuereinrichtung 34 weist ferner eine Eingabeeinheit 38 auf, durch die verschiedene Daten, wie beispielsweise eine abgeschätzte Gesamtmenge der in die Tankanlage 16 zu füllenden Milchcharge, Daten über den Milchproduzenten und Ähnliches, in die Steuereinrichtung 34 eingegeben werden können. Die Steuereinrichtung 34 kann ferner mit einem Vorgabedatenspeicher oder einer Funkanbindung verbunden sein.

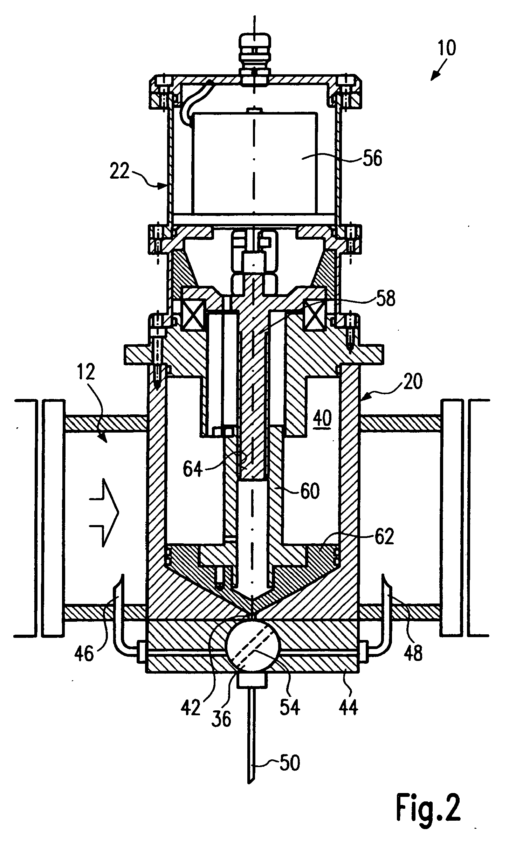

Der Zwischenbehälter 20, der nachfolgend unter Bezugnahme auf Fig. 2 näher erläutert wird, ist als Hohlzylinder ausgebildet und an seiner Oberseite offen. In die offene Oberseite des Zwischenbehälters 20 ist die Fördereinrichtung 22 eingesetzt und am Zwischenbehälter 20 befestigt. An dem dem offenen Ende entgegengesetzten Ende verjüngt sich der vom Zwischenbehälter 20 gebildete Innenraum 40 und mündet in eine Bohrung 42.

An der Unterseite des Zwischenbehälters 20 ist eine Rohrverzweigung 44 befestigt, in die das Mehrwegventil 36 integriert ist. Die Rohrverzweigung 44 weist eine in die Leitung 12 ragende Einströmleitung 46 sowie eine in Milchförderrichtung gesehen stromabwärts zur Einströmleitung 46 in die Leitung 12 mündende Rückführleitung 48 auf. Die beiden Leitungen 46 und 48 sind dabei so kurz wie möglich ausgebildet, um das Totvolumen des Leitungssystems möglichst gering zu halten.

Des Weiteren ist an der Unterseite der Rohrverzweigung 44 eine Kanüle 50 vorgesehen, die in den Probenbehälter 26 ragt, wenn der Probenbehälter 26 durch eine Hubeinrichtung 52 (vgl. Fig. 1) der Probeentnahmeeinrichtung 24 in eine Probeentnahmestellung bewegt ist. Unmittelbar gegenüber der Kanüle 50 mündet die Bohrung 42 des Zwischenbehälters 20 in den in die Rohrverzweigung 44 integrierten Ventilkörper des Mehrwegventils 36.

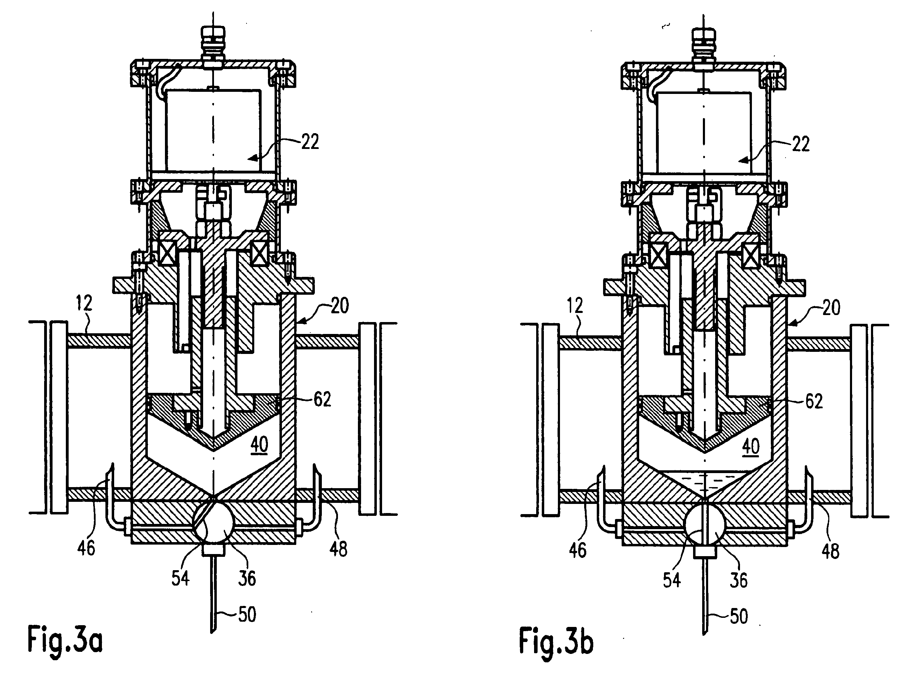

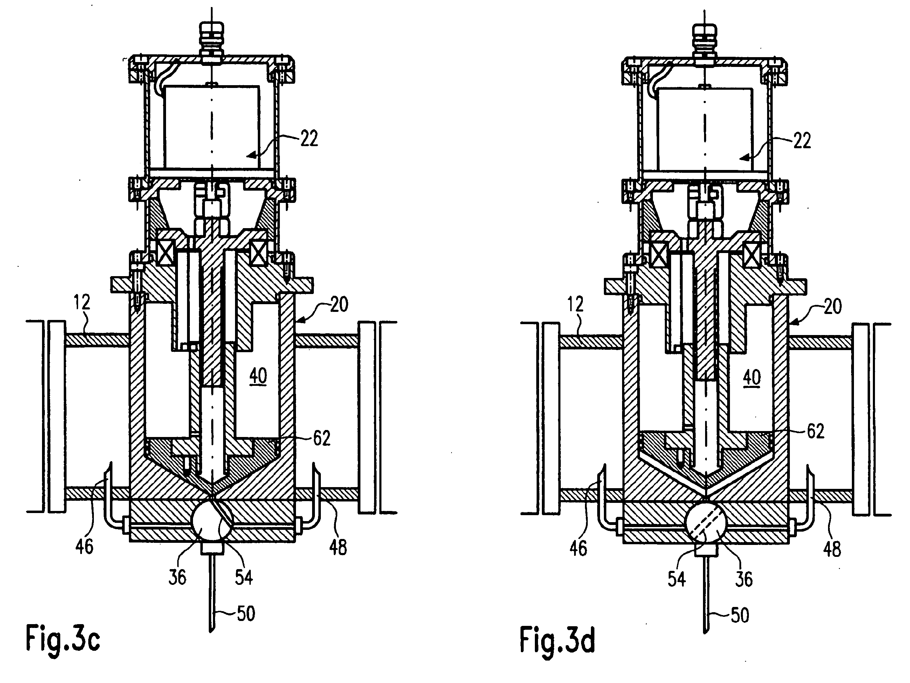

In dem Mehrwegventil 36 ist ein gestrichelt dargestellter Verbindungskanal 54 vorgesehen, der in einer ersten Ventilstellung des Mehrwegventils 36 (vgl. Fig. 3a) die Einströmleitung 46 mit dem Zwischenbehälter 20, in einer zweiten Ventilstellung (vgl. Fig. 3b) den Zwischenbehälter 20 mit der Kanüle 50 und in einer dritten Ventilstellung (vgl. Fig. 3c) den Zwischenbehälter 20 mit der Rückführleitung 48 verbindet. Des Weiteren kann das Mehrwegventil 36 in eine Zwischenstellung (vgl. Fig. 3d) zwischen den drei Ventilstellungen verstellt werden, in der die Rohrverzweigung 44 gesperrt ist.

Die Fördereinrichtung 22 weist einen mit der Steuereinrichtung 34 elektrisch leitend verbundenen Antrieb 56 auf, der mit einer in der Fördereinrichtung 22 drehbar gelagerten Spindel 58 gekuppelt ist. Die Spindel 58 steht mit einem in der Kolbenstange 60 eines Kolben 62 ausgebildeten Innengewinde 64 derart in Eingriff, dass bei einer Drehung der Spindel 58 der Kolben 62 innerhalb des Zwischenbehälters 20 in axialer Richtung angehoben und abgesenkt werden kann. Dabei eventuell entstehender Über- bzw. Unterdruck in den beiden vom Kolben 62 begrenzten Abschnitten im Innenraum 40 des Zwischenbehälters 20 wird durch die offene Strömungsverbindung zur Einströmleitung 46 bzw. zur Rückführleitung 48 sowie durch die offene Druckausgleichsleitung 28 ausgeglichen.

Nachfolgend wird die Funktionsweise der erfindungsgemäßen Vorrichtung unter Bezugnahme auf die Fig. 1 und 3a bis 3d näher erläutert.

Zunächst wird in einem ersten Schritt geschätzt, wie groß die Gesamtmenge der in die Tankanlage 16 zu fördernden Milchcharge ist. Anschließend wird die geschätzte Gesamtmenge der Milchcharge durch die Eingabeeinheit 38 in die Steuereinrichtung 34 eingegeben. In der Steuereinrichtung 34 wird auf Grundlage der geschätzten Gesamtmenge der Milchcharge und der vorgegebenen Probenmenge, die in den Probenbehälter 26 gefüllt werden soll, ein Proportionalitätsfaktor K bestimmt, der das Verhältnis zwischen der Gesamtmenge der Fluidcharge und einer in den Zwischenbehälter 20 zu füllenden Teilmenge der Fluidcharge vorgibt.

Dabei wird der Proportionalitätsfaktor K so vorgegeben, dass sich die aus der geschätzten Gesamtmenge der Milchcharge und dem Proportionalitätsfaktor K rechnerisch ergebende Teilmenge, die nach der Entnahme im Zwischenbehälter 20 theoretisch enthalten wäre, einerseits einem Vielfachen **n** der vorgegebenen Probenmenge von beispielsweise 20 bis 40 ml entspricht und andererseits um ein Mehrfaches **m** unter der in den Zwischenbehälter 20 füllbaren Maximalmenge liegt. Auf diese Weise wird sichergestellt, dass bei einer fehlerhaften Abschätzung der Gesamtmenge der Fluidcharge einerseits im Zwischenbehälter 20 eine so große Teilmenge enthalten ist, dass eine ordnungsgemäße Probenmenge entnommen werden kann, während andererseits gleichzeitig sichergestellt ist, dass ein Überfüllen des Zwischenbehälters 20 verhindert ist.

Nach Eingabe der geschätzten Gesamtmenge und Berechnung des Proportionalitätsfaktors K wird Milch durch eine Lanze 66 und einen Schlauch 68 aus einem Milchbehälter 70 in die Leitung 12 der Milchförderanordnung 14 gefördert. Sobald der Volumenstromsensor 18 erfasst, dass Milch durch die Leitung 12 strömt, gibt er ein entsprechendes Signal an die Steuereinrichtung 34 ab, die ihrerseits das Mehrwegventil 36 in die erste Ventilstellung (vgl. Fig. 3a) stellt, in der die Einströmleitung 46 mit dem Zwischenbehälter 20 verbunden ist. Gleichzeitig aktiviert die Steuereinrichtung 34 die Fördereinrichtung 22, die den Kolben 62 anhebt, so dass im Inneren 40 des Zwischenbehälters 20 ein Unterdruck entsteht, durch den Milch aus der Leitung 12 durch die Einströmleitung 46 in den Zwischenbehälter 20 gefördert wird. Ferner öffnet die Steuereinrichtung 34 das zweite Stellventil 32 der Druckausgleichsleitung 28. Da im Luftabschneider 30 ein Unterdruck von etwa 200 mbar herrscht, wird Luft aus dem Abschnitt oberhalb des Kolbens 62 abgeführt, so dass auch hier ein Unterdruck entsteht und die Hubbewegung des Kolbens 62 erleichtert ist.

Sobald ein bestimmtes Volumen an Milch im Zwischenbehälter 20 enthalten ist, das sich durch den Hub des Kolbens 62 ergibt, wird das Mehrwegventil 36 in seine dritte Ventilstellung (vgl. Fig. 3c) gestellt, in der der Zwischenbehälter 20 mit der Rückführleitung 48 in Strömungsverbindung steht. Anschließend wird die Fördereinrichtung 22 in entgegengesetzter Richtung betrieben, so dass sich der Kolben 62 wieder absenkt und die im Zwischenbehälter 20 enthaltene Milch aus der Rückführleitung 48 wieder in die Leitung 12 zurückdrückt. Gleichzeitig wird das erste Stellventil 29 geöffnet und das zweite Stellventil 32 geschlossen, so dass der Zwischenbehälter 20 zum Druckausgleich mit der Umgebung verbunden ist.

Durch diesen als Vorspülen bezeichneten Vorgang wird erreicht, dass eventuell im Zwischenbehälter 20 noch anhaftende Restmilch, die sich gegebenenfalls durch die zuvor angesaugte Milchcharge noch im Zwischenbehälter 20 befand, aus dem Zwischenbehälter 20 entfernt wird, und somit ein Verschleppen von Milch vermieden wird. Nach dem Vorspülen wird das Mehrwegventil 36 wieder in seine erste Ventilstellung (vgl. Fig. 3a) gestellt, so dass die Einströmleitung 46 erneut mit dem Zwischenbehälter 20 in Strömungsverbindung steht. Anschließend betätigt die Steuereinrichtung 34 die Fördereinrichtung 22 entsprechend dem vorgegebenen Proportionalitätsfaktor K, wobei kontinuierlich von dem Volumenstromsensor 18 der aktuelle Volumenstrom der Milchcharge durch die Leitung 12 erfasst wird, während der Zwischenbehälter 20 zum Druckausgleich wieder mit dem Luftabscheider 30 verbunden ist. Basierend auf dem aktuellen Volumenstrom und dem vorgegebenen Proportionalitätsfaktor K reguliert die Steuereinrichtung 34 die Förderleistung der Fördereinrichtung 22 so, dass eine zum aktuellen Volumenstrom proportionale Teilmenge in den Zwischenbehälter 22 gefördert wird.

Da durch das Vorspülen ein wenn auch geringer Teil des Volumenstroms beim Befüllen des Zwischenbehälters 20 nicht erfasst worden ist, erhöht die Steuereinrichtung 34 die Förderleistung der Fördereinrichtung 22 gegenüber dem vorgegebenen Proportionalitätsfaktor K nach Beginn des Befüllens des Zwischenbehälters 20 kurzzeitig so lange überproportional, bis die aus der vorbeiströmenden Milchcharge in den Zwischenbehälter 20 geförderte Menge zumindest annähernd zu dem Volumenstrom proportional ist, der die Einströmleitung 46 bei Erreichen der Proportionalität bereits passiert hat.

Sobald der Volumenstromsensor 18 erfasst, dass der Volumenstrom der Milchcharge einen vorgegebenen Grenzwert unterschreitet, d.h., dass größtenteils nur noch Luft in die Leitung 12 angesaugt wird, schließt die Steuereinrichtung 34 das Mehrwegventil 36 und sperrt die Rohrverzweigung 44, wie es in Fig. 3d gezeigt ist.

Anschließend aktiviert die Steuereinrichtung 34 die Fördereinrichtung 22, damit diese den Kolben 62 innerhalb des Zwischenbehälters 20 anhebt, während das zweite Steuerventil 32 zum Druckausgleich geöffnet ist. Des weiteren wird das Mehrwegventil 36 von der Steuereinrichtung 34 während des Anhebens des Kolbens 62 geschlossen, so dass im Innenraum 40 des Zwischenbehälters 20 ein Unterdruck entsteht. Sobald der Kolben 62 in seine angehobene Stellung bewegt ist, wird das Magnetventil 36 in seine zweite Ventilstellung bewegt, so dass Luft durch die Kanüle 50 in den Zwischenbehälter 20 angesaugt wird (vgl. Fig. 3b). Die durch die Kanüle 50 einströmende Luft durchdringt die Milch im Zwischenbehälter 20, wobei die einströmenden Luftblasen die Milch gleichmäßig und homogen durchmischen und dabei gleichzeitig belüften. Dieser Vorgang des Anhebens des Kolbens 62 und des anschließenden Öffnens des Magnetventils 36 wird solange wiederholt, bis die Milch im Zwischenbehälter 20 eine homogene Durchmischung zeigt.

Alternativ ist es auch möglich, den Kolben 62 kontinuierlich anzuheben, während gleichzeitig nach Erzeugen eines vorgegebenen Unterdrucks im Zwischenbehälter 20 das Magnetventil 36 geöffnet wird, so dass während des Anhebens des Kolbens 62 gleichzeitig Luft durch die Kanüle 50 in den Zwischenbehälter 20 einströmt.

Nach dem Durchmischen befindet sich im Zwischenbehälter 20 ein vorgegebenes Volumen aus Luft und Milch, wobei das Verhältnis zwischen homogen durchmischter Milch und Luft beispielsweise bei 1 : 1 liegt.

Um eine besonders gute Durchmischung der Milch zu erreichen, ist es besonders von Vorteil, wenn der Strömungsquerschnitt der Kanüle 50 möglichst klein ist, da auf diese Weise ein hoher Strömungswiderstand in der Kanüle 50 vorgegeben ist, durch den die Ausbildung besonders feiner Luftblasen beim Einströmen in die im Zwischenbehälter 20 enthaltene Milch verursacht wird, welche eine besonders gute Durchmischung der Milch bewirken. Die Luft sammelt sich nach dem Durchmischen der Milch oberhalb des Flüssigkeitsspiegels der Milch im Zwischenbehälter 20, wobei die Milch nach kurzer Zeit allenfalls noch geringe Mengen Luft enthält.

Nach dem Durchmischen und Belüften der Milch im Zwischenbehälter 20 sperrt das Mehrwegventil 36 (Fig. 3d) und die Steuereinrichtung 34 aktiviert die Probeentnahmeeinrichtung 24, so dass die Hubeinrichtung 52 der Probeentnahmeeinrichtung 24 den Probenbehälter 26 soweit anhebt, bis die Kanüle 50 in den Probenbehälter 26 hineinragt, wobei eine gegebenenfalls an der Öffnung des Probenbehälters 26 vorgesehene geschlitzte Gummimembran von der Kanüle 50 durchstoßen wird.

Anschließend wird das Mehrwegventil 36 in die zweite Ventilstellung gestellt (vgl. Fig. 3b), in der der Zwischenbehälter 20 mit der Kanüle 50 in Strömungsverbindung, und das zweite Steuerventil 32 geschlossen, während das erste Steuerventil 29 zum atmosphärischen Druckausgleich geöffnet wird. Danach aktiviert die Steuereinrichtung 34 die Fördereinrichtung 22, so dass der Kolben 62 um eine vorgegebene Wegstrecke nach unten bewegt und die vorgegebene Probenmenge weitestgehend luftfreier Milch durch die Kanüle 50 in den Probenbehälter 26 gedrückt wird. Schließlich wird der Probenbehälter 26 zur Entnahme aus der Probeentnahmeeinrichtung 24 abgesenkt.

Die Fördereinrichtung 22 erfüllt bei der erfindungsgemäßen Vorrichtung 10 also drei verschiedene Funktionen. Einerseits dient die Fördereinrichtung 22 als volumenstromproportionale Kolbenpumpe, mit der eine Teilmenge aus dem vorbeiströmenden Fluidstrom volumenstromproportional in den Zwischenbehälter 20 gefördert werden kann. Des Weiteren dient die Fördereinrichtung 22 als Mischeinrichtung zum homogenen Durchmischen der im Zwischenbehälter 20 enthaltenen Milch. Ferner arbeitet die Fördereinrichtung 22 als Dosierpumpe, mit der eine definierte, vorgegebene Probenmenge in den Probenbehälter 26 abgegeben werden kann.

Nach der Entnahme der Probe wird das Magnetventil 36 zunächst geschlossen und der Kolben 62 nach oben bewegt, damit im Innenraum 40 des Zwischenbehälters 20 ein Unterdruck entsteht. Sobald sich der Kolben 62 in seiner angehobenen Stellung befindet, wird das Magnetventil 36 in seiner zweite Ventilstellung bewegt, in der es mit der Kanüle 50 in Strömungsverbindung steht, wodurch die noch in der Kanüle 50 befindliche Milch in den Zwischenbehälter 20 gesaugt wird. Dieser Vorgang kann mehrfach wiederholt werden, bis sichergestellt ist, dass die Kanüle 50 gereinigt ist.

Bei nach oben bewegtem Kolben 62 wird anschließend das Magnetventil 36 in seine dritte Ventilstellung (vgl. Fig. 3c) gestellt, in der es mit der Rückführleitung 48 in Strömungsverbindung steht. Danach wird der Kolben 62 in seine Endstellung bewegt, in der er an der mit seiner kegelförmigen Stirnseite am hohlkegelförmigen Ende des Zwischenbehälters 20 anliegt. Hierdurch wird die Restmilch im Zwischenbehälter 20 in die Leitung 12 zurückgefördert, aus der sie in die Tankanlage 16 abgesaugt wird.

Zum anschließenden Reinigen der Einströmleitungen 46 von Restmilch wird das Magnetventil 36 in seine erste Ventilstellung gestellt und der Kolben 62 angehoben, so dass Luft aus der Leitung 12 durch die Einströmleitung 46 in den Zwischenbehälter 20 einströmt und dabei eventuell noch in der Einströmleitung 46 anhaftende Milchreste mitreißt. Anschließend wird das Magnetventil 36 in seine dritte Ventilstellung gestellt und der Kolben 62 wieder abwärts bewegt, wobei die im Zwischenbehälter 20 enthaltene Luft aus dem Zwischenbehälter 20 durch die Rückführleitung 48 in die Leitung 12 gedrückt wird und dabei eventuell noch in der Rückführleitung 48 anhaftende Milchreste mitreißt.

## Patentansprüche

1. Verfahren zur Entnahme einer Probe aus einer Fluidcharge, insbesondere zur Entnahme einer repräsentativen Probe aus einer Milchcharge, bei dem
a) an einer Entnahmestelle aus der vorbeiströmenden Fluidcharge entsprechend einem vorgebbaren Proportionalitätsfaktor (K) eine Teilmenge der Fluidcharge in einen Zwischenbehälter (20) gefördert wird,
b) aus der Teilmenge im Zwischenbehälter (20) eine vorgegebene Probenmenge der Fluidcharge entnommen wird,
c) der Zwischenbehälter (20) nach der Probenentnahme entleert wird, und
d) der aktuelle Volumenstrom der Fluidcharge vor der Entnahmestelle der Teilmenge, welche vor einer Luftabscheidung vorgesehen ist, erfaßt wird,
e) die Teilmenge entsprechend dem vorgebbaren Proportionalitätsfaktor (K) zumindest annähernd proportional zu dem erfaßten aktuellen Volumenstrom der Fluidcharge in den Zwischenbehälter (20) gefördert wird,
f) die im Zwischenbehälter (20) enthaltene Teilmenge der Fluidcharge vor der Probenentnahme durchmischt wird,
g) zum Durchmischen der Teilmenge im Zwischenbehälter (20) aufeinanderfolgend ein Unterdruck erzeugt und der Unterdruck anschließend ausgeglichen wird,
h) der Unterdruck mittels Kolbenbewegung im Zwischenbehälter (20) erzeugt wird, und
i) die Probe nach dem Durchmischen bei der Entnahme aus dem Zwischenbehälter (20) direkt in einen Probenbehälter (26) abgegeben wird.

2. Verfahren nach Anspruch 1,
**dadurch gekennzeichnet,**
**dass** der Zwischenbehälter (20) vor dem Befüllen mit der Teilmenge zunächst durch Fluid aus der Fluidcharge, aus der die Probe nachfolgend entnommen werden soll, vorgespült wird.

3. Verfahren nach Anspruch 2,
**dadurch gekennzeichnet,**
**dass** die Förderleistung, mit der die Teilmenge nach dem Vorspülen in den Zwischenbehälter (20) gefördert wird, gegenüber der zum aktuellen Volumenstrom proportionalen Förderleistung kurzzeitig solange überproportional erhöht wird, bis die aus der an der Entnahmestelle vorbeiströmenden Fluidcharge in den Zwischenbehälter (20) geförderte Menge zumindest annähernd zu dem Volumenstrom proportional ist, der die Entnahmestelle bei Erreichen der Proportionalität bereits passiert hat.

4. Verfahren nach einem der vorhergehenden Ansprüche,
**dadurch gekennzeichnet,**
**dass** im Zwischenbehälter (20) zum Entleeren der nach der Probenentnahme im Zwischenbehälter (20) enthaltenen Restmenge der Fluidcharge Überdruck erzeugt wird.

5. Verfahren nach einem der vorhergehenden Ansprüche,
**dadurch gekennzeichnet,**
**dass** nach der Probenentnahme und/oder nach dem Entleeren des Zwischenbehälters (20) in diesem ein Unterdruck erzeugt wird, durch den ein Reinigungsgas, insbesondere Luft, durch mit dem Zwischenbehälter (20) in Verbindung stehende Leitungen (46, 50) in den Zwischenbehälter (20) gesaugt wird.

6. Verfahren nach Anspruch 5,
**dadurch gekennzeichnet,**
**dass** nach dem Ansaugen des Reinigungsgases in den Zwischenbehälter (20) in diesem ein Überdruck erzeugt und das Reinigungsgas durch eine mit dem Zwischenbehälter (20) verbundene Rückführleitung (48) abgeleitet wird.

7. Verfahren nach einem der Ansprüche 1 bis 6,
**dadurch gekennzeichnet,**
**dass** der Innendruck im Zwischenbehälter (20) zumindest während des Befüllens mit der Teilmenge ausgeglichen wird.

8. Verfahren nach einem der Ansprüche 1 bis 7,
**dadurch gekennzeichnet,**
**dass** der zum Durchmischen erzeugte Unterdruck stufenweise erzeugt wird,
**dass** die Teilmenge vor der Probenentnahme zumindest annähernd homogen durchmischt wird, und
die Entnahme der Probe unmittelbar nach dem Durchmischen erfolgt.

9. Verfahren nach einem der Ansprüche 1 bis 8,
**dadurch gekennzeichnet,**
**dass** der Proportionalitätsfaktor (K) so vorgegeben wird, dass die Teilmenge größer oder gleich der vorgegebenen Probenmenge ist.

10. Verfahren nach einem der Ansprüche 1 bis 9,
**dadurch gekennzeichnet,**
- **dass** die Gesamtmenge der Fluidcharge, aus der die Probe entnommen werden soll, geschätzt wird,
- **dass** der vorgebbare Proportionalitätsfaktor (K) aus der geschätzten Gesamtmenge der Fluidcharge und der vorgegebenen Probenmenge bestimmt wird, und
- **dass** der vorgebbare Proportionalitätsfaktor (K) so bestimmt wird, dass die sich aus der geschätzten Gesamtmenge und dem Proportionalitätsfaktor (K) rechnerisch ergebende Teilmenge einem Vielfachen (n) der vorgegebenen Probenmenge entspricht und um ein Mehrfaches (m) unter der in den Zwischenbehälter (20) füllbaren Maximalmenge liegt.

11. Verfahren nach einem der Ansprüche 1 bis 10,
**dadurch gekennzeichnet,**
**dass** das Fluid eine schäumende Flüssigkeit und/oder eine Emulsion, insbesondere Milch, ist.

12. Vorrichtung zur Entnahme einer Probe aus einer Fluid-charge, insbesondere zur Durchführung des Verfahrens nach einem der Ansprüche 1 bis 11, mit
a) einem Zwischenbehälter (20), der mit einer Leitung (12) für die Fluidcharge verbindbar ist,
b) einer Fördereinrichtung (22), durch die der Zwischenbehälter (20) mit einer Teilmenge aus der durch die Leitung (12) strömenden Fluidcharge befüllbar ist,
c) einer Probeentnahmeeinrichtung (24), die mit dem Zwischenbehälter (20) in Strömungsverbindung steht, wobei,
d) mindestens ein Volumenstromsensor (18) zum Erfassen des aktuellen Volumenstroms der Fluidcharge in der Leitung (12) vorgesehen ist,
e) eine mit dem Volumenstromsensor (18) und der Fördereinrichtung (22) verbundene Steuereinrichtung (34) zur volumenstromproportionalen Einstellung der von der Fördereinrichtung (22) in den Zwischenbehälter (20) zu fördernden Teilmenge vorgesehen ist,
f) die Fördereinrichtung (22) zumindest teilweise in den Zwischenbehälter (20) integriert ist und durch sie im Zwischenbehälter (20) ein Unterdruck und ein Überdruck erzeugbar ist, und
g) die Fördereinrichtung (22) einen im Zwischenbehälter (20) beweglichen Kolben (62) aufweist, wobei die aus dem Zwischenbehälter (20) abgebbare Probenmenge proportional zum Hub des Kolbens (62) ist.

13. Vorrichtung nach Anspruch 12,
**dadurch gekennzeichnet,**
- **dass** am Zwischenbehälter (20) eine in einen Luftabscheider (30) mündende Druckausgleichsleitung (28) vorgesehen ist und
- **dass** die Druckausgleichsleitung (28) durch ein von der Steuereinrichtung (34) ansteuerbares Stellventil (32) absperrbar ist.

14. Vorrichtung nach einem der Ansprüche 12 bis 13,
**dadurch gekennzeichnet,**
- **dass** der Zwischenbehälter (20) und die Probeentnahmeeinrichtung (24) durch eine gemeinsame absperrbare Rohrverzweigung (44) miteinander in Strömungsverbindung stehen und
- **dass** die Rohrverzweigung (44) eine Einströmleitung (46) und eine Rückführleitung (48) aufweist, die mit der Leitung (12) für die Fluidcharge verbindbar sind.

15. Vorrichtung nach Anspruch 14,
**dadurch gekennzeichnet,**
**dass** in der Rohrverzweigung (44) ein von der Steuereinrichtung betätigbares Mehrwegventil (36) vorgesehen ist, das in einer ersten Ventilstellung die Einströmleitung (46) mit dem Zwischenbehälter (20), in einer zweiten Ventilstellung den Zwischenbehälter (20) mit der Probeentnahmeeinrichtung (24) und in einer dritten Ventilstellung den Zwischenbehälter (20) mit der Rückführleitung (48) verbindet, und das in zumindest einer Zwischenstellung zwischen den drei Ventilstellungen die Rohrverzweigung (44) sperrt.

16. Vorrichtung nach einem der Ansprüche 12 bis 15
**dadurch gekennzeichnet,**
**dass** der Zwischenbehälter (20) und/oder der Kolben (62) zumindest an den Abschnitten, die mit dem Fluid in Kontakt kommen können, hydrophobe und/oder polierte Oberflächen aufweisen bzw. aufweist.

## Claims

1. Method for removing a sample from a fluid batch, particularly for removing a representative sample from a milk batch, in which
a) at a removal point a partial quantity of the fluid batch which is flowing by and corresponding to a predeterminable proportionality factor (K) is delivered into an intermediate container (20),
b) from the partial quantity in the intermediate container (20) a predetermined sample quantity of the fluid batch is removed,
c) the intermediate container (20) is emptied after sampling and
d) the present volume flow of the fluid batch is determined upstream of the partial quantity removal point which is upstream of an air separation,
e) the partial quantity is delivered into the intermediate container (20) corresponding to the predeterminable proportionality factor (K) and at least approximately proportionally to the detetermined, present fluid batch volume flow,
f) the partial quantity of the fluid batch contained in the intermediate container (20) is mixed prior to sampling,
g) for the mixing of the partial quantity in the intermediate container (20) successively a vacuum is produced and the vacuum is then compensated,
h) the vacuum is produced by a piston movement in the intermediate container (20) and
i) following mixing and on removal from the intermediate container (20), the sample is supplied directly to a sample container (26).

2. Method according to claim 1,
**characterized in that**,
prior to filling with the partial quantity, the intermediate container (20) is prerinsed initially by fluid from the fluid batch from which the sample is to be subsequently taken.

3. Method according to claim 2,
**characterized in that**
the delivery rate with which the partial quantity is delivered following prerinsing in the intermediate container (20) is overproportionally increased for a short period compared with the delivery rate proportional to the present volume flow until the quantity delivered into the intermediate container (20) from the fluid batch flowing past the removal point is at least approximately proportional to the volume flow which has already passed the removal point on reaching proportionality.

4. Method according to one of the preceding claims,
**characterized in that**
an overpressure is produced in the intermediate container (20) for emptying the residual quantity of the fluid batch contained in said intermediate container (20) following sampling.

5. Method according to one of the preceding claims,
**characterized in that**
following sampling and/or emptying of the intermediate container (20) a vacuum is produced in the latter through which a cleaning gas, particularly air, is sucked into the intermediate container (20) through lines (46, 50) connected thereto.

6. Method according to claim 5,
**characterized in that**
following the suction of the cleaning gas into the intermediate container (20), an overpressure is produced therein and the cleaning gas is led off through a return line (48) connected to the intermediate container (20).

7. Method according to one of the claims 1 to 6,
**characterized in that**
the internal pressure of the intermediate container (20) is compensated at least during the filling with the partial quantity.

8. Method according to one of the claims 1 to 7,
**characterized in that**
the vacuum produced for mixing purposes is progressively produced,
that the partial quantity is at least approximately homogeneously mixed prior to sampling and
the removal of the sample takes place directly following mixing.

9. Method according to one of the claims 1 to 8,
**characterized in that**
the proportionality factor (K) is predetermined in such a way that the partial quantity is equal to or greater than the predetermined sample quantity.

10. Method according to one of the claims 1 to 9,
**characterized in that**
- the total quantity of the fluid batch from which the sample is to be taken is estimated,
- the predeterminable proportionality factor (K) is determined from the estimated fluid batch total quantity and the predetermined sample quantity and
- that the predeterminable proportionality factor (K) is so determined that the partial quantity mathematically established from the estimated total quantity and the proportionality factor (K) corresponds to a multiple (n) of the predetermined sample quantity and is lower by a multiple (m) than the maximum quantity which can be filled into the intermediate container (20).

11. Method according to one of the claims 1 to 10,
**characterized in that**
the fluid is a frothing liquid and/or an emulsion, particularly milk.

12. Apparatus for removing a sample from a fluid batch, particularly for performing the method according to one of the claims 1 to 11, having
a) an intermediate container (20) connectable to a line (12) for the fluid batch,
b) a delivery device (22) through which the intermediate container (20) can be filled with a partial quantity of the fluid batch flowing through the line (12),
c) a sampling device (24) in flow connection with the intermediate container (20), wherein
d) at least one volume flow sensor (18) is provided for determining the present volume flow of the fluid batch in the line (12),
e) a control device (34) connected to the volume flow sensor (18) and the delivery device (22) is provided for the volume flow-proportional setting of the partial quantity to be delivered by delivery device (22) into the intermediate container (20),
f) the delivery device (22) is at least partly integrated into the intermediate container (20) and through it a vacuum and an overpressure can be produced in the intermediate container (20) and
g) the delivery device (22) has a piston (62) movable in the intermediate container (20) and the sample quantity which can be supplied by intermediate container (20) is proportional to the stroke of the piston (62).

13. Apparatus according to claim 12,
**characterized in that**
- on intermediate container (20) is provided a pressure compensating line (28) issuing into an air separator (30) and
- the pressure compensating line (28) can be shut off by a servovalve (32) controllable by control device (34).

14. Apparatus according to one of the claims 12 and 13,
**characterized in that**
- the intermediate container (20) and the sampling device (24) are in flow connection with one another through a common, blockable pipe branch (44) and
- the pipe branch (44) has an inflow line (46) and a return line (48) connectable to the fluid batch line (12).

15. Apparatus according to claim 14,
**characterized in that**
in the pipe branch (44) is provided a multiway valve (36) operable by the control device and which in a first valve position connects the inflow line (46) to the intermediate container (20), in a second valve position connects the intermediate container (20) to the sampling device (24) and in a third valve position connects the intermediate container (20) to the return line (48) and which in at least one intermediate position between the three valve positions blocks the pipe branch (44).

16. Apparatus according to one of the claims 12 to 15,
**characterized in that**
at least in the sections which can come into contact with the fluid, the intermediate container (20) and/or piston (62) have hydrophobic and/or polished surfaces.

## Revendications

1. Procédé de prélèvement d'un échantillon depuis une charge de fluide, en particulier de prélèvement d'un échantillon représentatif d'une charge de lait, dans lequel
a) en un point de prélèvement, une quantité partielle de la charge de fluide est envoyée dans un récipient intermédiaire (20) depuis la charge de fluide en écoulement, suivant un facteur de proportionnalité (K) prédéfinissable,
b) à partir de la quantité partielle, une quantité prédéterminée d'échantillon de la charge de fluide est prélevée dans le récipient intermédiaire (20),
c) le récipient intermédiaire (20) est vidé après le prélèvement d'échantillon, et
d) le débit volumique réel de la charge de fluide avant le point de prélèvement de la quantité partielle, qui est prévu avant une séparation d'air, est relevé,
e) la quantité partielle est envoyée dans le récipient intermédiaire (20) conformément au facteur de proportionnalité prédéfinissable (K) d'une façon au moins approximativement proportionnelle au débit volumique réel relevé de la charge de fluide,
f) la quantité partielle de la charge de fluide contenue dans le récipient intermédiaire (20) est mélangée avant le prélèvement d'échantillon,
g) pour mélanger la quantité partielle dans le récipient intermédiaire (20), on produit successivement une dépression et on compense ensuite la dépression,
h) la dépression est produite par un mouvement de piston dans le récipient intermédiaire (20), et
i) lors du prélèvement depuis le récipient intermédiaire (20), l'échantillon est délivré, après le mélange, directement dans un récipient à échantillon (26).

2. Procédé selon la revendication 1, ***caractérisé en ce qu*'**avant le remplissage avec la quantité partielle, le récipient intermédiaire (20) est d'abord pré-rincé avec du fluide provenant de la charge de fluide depuis laquelle l'échantillon doit ensuite être prélevé.

3. Procédé selon la revendication 2, ***caractérisé en ce que*** la puissance d'acheminement par laquelle la quantité partielle est envoyée dans le récipient intermédiaire (20) après le pré-rinçage est augmentée brièvement de manière disproportionnée par rapport à la puissance d'acheminement proportionnelle au débit volumique réel, jusqu'à ce que la quantité transportée dans le récipient intermédiaire (20) depuis la charge de fluide s'écoulant au point de décharge soit au moins approximativement proportionnelle au débit volumique qui est passé au point de prélèvement quand on atteint la proportionnalité.

4. Procédé selon l'une quelconque des revendications précédentes, ***caractérisé en ce qu*'**une surpression est produite dans le récipient intermédiaire (20) afin de vider la quantité résiduelle de la charge de fluide contenue dans le récipient intermédiaire (20) après le prélèvement d'échantillon.

5. Procédé selon l'une quelconque des revendications précédentes, ***caractérisé en ce qu*'**après le prélèvement d'échantillon et/ou après la vidange du récipient intermédiaire (20), une dépression est produite dans celui-ci, par laquelle un gaz de nettoyage, en particulier de l'air, est aspiré dans le récipient intermédiaire (20) par des conduites (46, 50) communiquant avec le récipient intermédiaire (20).

6. Procédé selon la revendication 5, ***caractérisé en ce qu*'**après l'aspiration du gaz de nettoyage dans le récipient intermédiaire (20), une surpression est produite dans celui-ci et le gaz de nettoyage est évacué par une conduite de retour (48) communiquant avec le récipient intermédiaire (20).

7. Procédé selon l'une quelconque des revendications 1 à 6, ***caractérisé en ce que*** la pression intérieure dans le récipient intermédiaire (20) est compensée avec la quantité partielle, au moins pendant le remplissage.

8. Procédé selon l'une quelconque des revendications 1 à 7, ***caractérisé***
***en ce que*** la dépression produite pour le mélange est produite progressivement,
***en ce que*** la quantité partielle est mélangée de façon au moins approximativement homogène avant le prélèvement d'échantillon, et
le prélèvement de l'échantillon a lieu immédiatement après le mélange.

9. Procédé selon l'une quelconque des revendications 1 à 8, ***caractérisé en ce que*** le facteur de proportionnalité (K) est choisi de telle sorte que la quantité partielle soit supérieure ou égale à la quantité d'échantillon prédéterminée.

10. Procédé selon l'une quelconque des revendications 1 à 9, ***caractérisé***
- ***en ce que*** la quantité totale de la charge de fluide dont doit être prélevé l'échantillon est estimée,
- ***en ce que*** le facteur de proportionnalité prédéfinissable (K) est déterminé à partir de la quantité totale estimée de la charge de fluide et de la quantité échantillon prédéterminée, et
- ***en ce que*** le facteur de proportionnalité (K) prédéfinissable est déterminé de telle sorte que la quantité partielle résultant par calcul de la quantité totale estimée et du facteur de proportionnalité (K) correspond à un multiple (n) de la quantité échantillon prédéterminée et est inférieure d'un multiple (m) à la quantité maximale pouvant être placée dans le récipient intermédiaire (20).

11. Procédé selon l'une quelconque des revendications 1 à 10, ***caractérisé en ce que*** le fluide est un liquide moussant et/ou une émulsion, en particulier du lait.

12. Dispositif pour prélever un échantillon depuis une charge de fluide, en particulier pour appliquer le procédé selon l'une quelconque des revendications 1 à 11, avec
a) un récipient intermédiaire (20) qui peut être relié à une conduite (12) pour la charge de fluide,
b) un dispositif de transport (22) par lequel le récipient intermédiaire (20) peut être rempli avec une quantité partielle de la charge de fluide s'écoulant à travers la conduite (12),
c) un dispositif (24) de prélèvement d'échantillon qui est en liaison d'écoulement avec le récipient intermédiaire (20),
d) dans lequel au moins un capteur (18) de débit volumique est prévu pour relever le débit volumique réel de la charge de fluide dans la conduite (12),
e) il est prévu un dispositif de commande (34) relié au capteur (18) de débit volumique et au dispositif de transport (22) pour ajuster de manière proportionnelle au débit volumique la quantité partielle devant être envoyée dans le récipient intermédiaire (20) par le dispositif de transport (22),
f) le dispositif de transport (22) est intégré au moins partiellement dans le récipient intermédiaire (20) et une dépression et une surpression peuvent être produites par lui dans le récipient intermédiaire (20), et
g) le dispositif de transport (22) comprend un piston (62) mobile dans le récipient intermédiaire (20), la quantité d'échantillon pouvant être délivrée depuis le récipient intermédiaire (20) étant proportionnelle à la course du piston (62).

13. Dispositif selon la revendication 12, ***caractérisé***
- ***en ce qu***'il est prévu sur le récipient intermédiaire (20) une conduite (28) d'équilibrage de pression débouchant dans un séparateur (30) d'air, et
- ***en ce que*** la conduite (28) d'équilibrage de pression peut être fermée par une vanne (32) pouvant être commandée par le dispositif de commande (34).

14. Dispositif selon l'une quelconque des revendications 12 à 13, ***caractérisé***
- ***en ce que*** le récipient intermédiaire (20) et le dispositif (24) de prélèvement d'échantillon sont mutuellement en liaison d'écoulement par une ramification (44) de conduite pouvant être fermée en commun, et
- ***en ce que*** la ramification (44) de conduite présente une conduite (46) d'arrivée et une conduite (48) de retour qui peuvent être reliées à la conduite (12) pour la charge de fluide.

15. Dispositif selon la revendication 14, ***caractérisé en ce que*** dans la ramification (44) de conduite est prévue une vanne multivoie (36) actionnable par le dispositif de commande, qui relie dans une première position la conduite d'arrivée (46) au récipient intermédiaire (20), dans une deuxième position le récipient intermédiaire (20) au dispositif (24) de prélèvement d'échantillon et dans une troisième position le récipient intermédiaire (20) à la conduite de retour (48), et qui, dans au moins une position intermédiaire entre les trois positions de vanne, ferme la ramification (44) de conduite.

16. Dispositif selon l'une quelconque des revendications 12 à 15, ***caractérisé en ce que*** le récipient intermédiaire (20) et/ou le piston (62) présente(nt) des surfaces hydrophobes et/ou polies au moins sur les sections qui entrent en contact avec le fluide.
